# EUROPEAN PATENT SPECIFICATION

(11) **EP 0 580 897 B1**
(45) Date of publication and mention of the grant of the patent: **15.01.1997**
(21) Application number: 92121293.2
(22) Date of filing: 15.12.1992
(51) Int. Cl.: B05B 11/02

(54) **Single-dose nasal dispenser for atomized liquid drugs**
Nasenspender zum Versprühen einer einmaligen Dosis von flüssigen Arzneimittel
Pulvérisateur nasal d'une dose unique de médicament liquide

(30) Priority: 03.06.1992 IT MI921370
(43) Date of publication of application: 02.02.1994
(73) Proprietor: ELETTRO PLASTICA S.p.A., I-20089 Quinto de Stampi-Rozzano (Milano) (IT)
(72) Inventor: Marelli, Andrea, I-20089 Rozzano (MI) (IT)
(74) Representative: Giambrocono, Alfonso, Dr. Ing.

(56) References cited:
- EP-A- 0 021 123
- EP-A- 0 201 701
- EP-A- 0 212 188
- EP-A- 0 311 863
- EP-A- 0 486 894
- WO-A-93/00172

## Description

The invention relates to a manually actuable dispenser for dispensing dosed amounts of liquid drugs in atomized form according to preamble of claim 1 and as known from EP-A-201 701.

It is known that many drugs, for instance calcitonin, are nasally administered: since these drugs are extremely expensive and sometimes they can cause undesired side effects if they are sprayed into nose in exceeding doses, some devices are used for their application by which an exactly predetermined liquid amount, in a totally atomized form, can be dispensed, leaving the lowest residual possible of said liquid unused in said devices.

Indeed, it often happens that the known devices are not suitable for dispensing practically all the liquid substance therein retained, or that the dispensing occurs also at a relatively low pressure, with consequent dropping (and therefore loss) of said liquid outside of the dispensing nozzle of the nasal distributor, or further that the devices have a very complicated structure and therefore extremely expensive, to such an extent to suggest sometimes, at least in part, their re-use.

Devices of such a type are, for instance, described in the patents EP-B-0218840 corresponding to the US-A-4.921.142 patent, and in the improvement of said last patent, depicted in the EP-A-0388651 and in the PCT/WO/91/13689 (PCT/EP91/00457 application) patents, besides that in the EP-A-0452728 patent.

The WO-A 9 300 172 discloses a spray dispenser in which sealing at rest is assured by the free edge of a piston cooperating with a seat provided on an enlarged head of a movable stem upon which the piston is slidably mounted. At rest, the surface of the piston exposed the liquid contained within the dispenser is relatively small and therefore also the force exerted thereon by the liquid when the dispenser is actuated is small: the consequence is that the force pushing the piston to seal on said enlarged head must be relatively small in order to enable actuation of the dispenser with the finger of an hand. Therefore, the pressure of the liquid sprayed through the nozzle of the dispenser is necessarily low on opening and closure and such liquid will drip out especially at the beginning and at the end of the delivery. Moreover, in order to prevent losses of liquid from the nozzle when the disper is at rest, a spring or other elastic system urging the piston free edge against the stem seat should be provided, thus increasing the cost of the dispenser.

The EP-A-0 201 701 and EP-A- 0 212 188 disclose dispensers whose structures are very complex and expensive and in which sealing at rest is assured by an enlarged head of a stem pushed by a spring against a collar or a sleeve which is slidably mounted on said stem. When the dispensers are actuated said head is lowered in respect of the sleeve which initially remains standing: consequently opening the emission channel takes place slowly and the liquid can be delivered even at extremely low pressure, surely dripping from the outlet nozzle at least at delivery beginning and stopping.

All the above mentioned patents describe nasal dispensers having a rather complicated structure and therefore expensive, some of which, at the end of their use, keeping in their inside a remarkable amount of the liquid, others not assuring a sufficient seal and insulation of said liquid in the inside of the device during the storage, further, others allowing the liquid dispensing even at excessively low dispensing pressures, with consequent liquid dropping.

To simplify the structure and to therefore control the expenditures of such devices, the French patent FR-B-2625981 proposed a nasal dispensing device comprising a seat in which a piston, which is part of the device itself, extends, in said seat a cylindrical ampoule being insertable which holds the desired amount of the liquid: when said ampoule is inserted in the mentioned seat, the piston, which is part of the device, penetrates in the inside of the ampoule itself, thus allowing the expulsion of the liquid in atomized form when said ampoule is pushed inside its own seat.

The main purpose of the present invention is that one to realize a nasal dispenser of the above cited type, comprising a container of a single dose of a liquid having pharmaceutical activity, manually actuable for dispensing practically totally such a dose of the liquid, at a dispensing pressure always higher than a minimum predetermined value.

Another purpose of the invention is that one to realize a nasal dispenser of the above mentioned type which assures, before manually actuating said device, a perfect seal and insulation of the liquid therein contained, the dispenser having, besides, a very simple structure and being extremely cheap without the presence of any additional spring biasing means and such that no axial movement of the stem relative to the sleeve is necessary in order to dispense the liquid.

The above purposes are achieved by a manually actuated nasal dispenser for dispensing dosed amounts of atomized liquid drugs, comprising a first body (1) having a cylindrical chamber (2) for containing at least one liquid drug dose and a second body (3) slidingly mounted on said first body and having a nozzle (12) for drug dispensing, said first body (1) having an elongated shape and delimiting said chamber which is open at one end of said first body itself whose other end has a seat (4) for the resting of a finger of one hand, and said second body (3) consisting of a hollow elongated element (3A, 3B) of rounded shape from which at least one shaped element (5) externally extends for the resting of at least another finger of said hand and into which a tubular wall (6) extends to which a piston slidable within said chamber (2) is integral, said nozzle (12) being provided in said hollow element (3A) in correspondence of a free end thereof, one end of an elongated cylindrical stem (7) being inserted and retained within said tubular wall (6), said stem (7) extending beyond the free edge of said tubular wall (6) and having its other end extending into said chamber (2), said piston comprising a tubular sleeve (8, 9, 10) made of resiliently yielding material superimposed to said stem (7), one end portion (9) of said sleeve being mounted on said tubular wall (6) while the other end portion (10) of the same sleeve extends and is seal slidable in said chamber (2), the intermediate portion of said sleeve (8, 9, 10) consisting of a continuos wall (8) resiliently flexible, characterized in that said end portion (9) of the sleeve (8, 9, 10) mounted on the tubular wall (6) is restrained thereon, and in that at least one annular continuous rib (11) projects from said continuous wall (8) towards the inside, said rib (11) being resiliently pushed on the outer cylindrical surface of said stem (7) by the resilient action of said continuous wall (8) to seal on it in rest condition of the dispenser, at least one continuous passage being formed between the internal surface of said sleeve (8, 9, 10) and respectively the external surface of said stem (7), such passage being open at its ends in correspondence of said nozzle (12) and respectively of said chamber (2) and being seal intercepted, in an intermediate position, by said rib (11) protruding from said intermediate portion (8) of said sleeve, said rib being moved away from said stem surface when liquid pressure in said chamber due to actuation by the user causes said continuous wall (8) to be outwardly deformed.

Conveniently, said first and second bodies (1, 3) have shaped portions (24, 25) cooperating among them to prevent the free end of said stem and of said sleeve from disconnecting from said chamber (2) and retaining means (21, 24, 25) are provided which can be snap-passed upon manual actuaction of said dispenser for retaining said first and second bodies (1, 3) in the resting position preliminary to dispensing.

A preferred embodiment of the invention shown by mere and non limiting example will be now described to make the structure and the features of the nasal dispenser according to the present invention clearer, reference being made to the accompanying drawing wherein:
- Figure 1 is an enlarged cross-section, of a single dose nasal dispenser according to the invention whereof
- Figures 2 and 3 respectively represent, in frontal elevation, the end portion in two different blocked positions of the two dispenser components which are movable one upon the other.

The dispenser illustrated in the drawings comprises a first body 1 which defines a cylindrical chamber 2 suitable for containing a dose of a liquid drug, and a second body 3 slidably mounted on said body 1 by manual action, such bodies being preferably made of plastics, by molding.

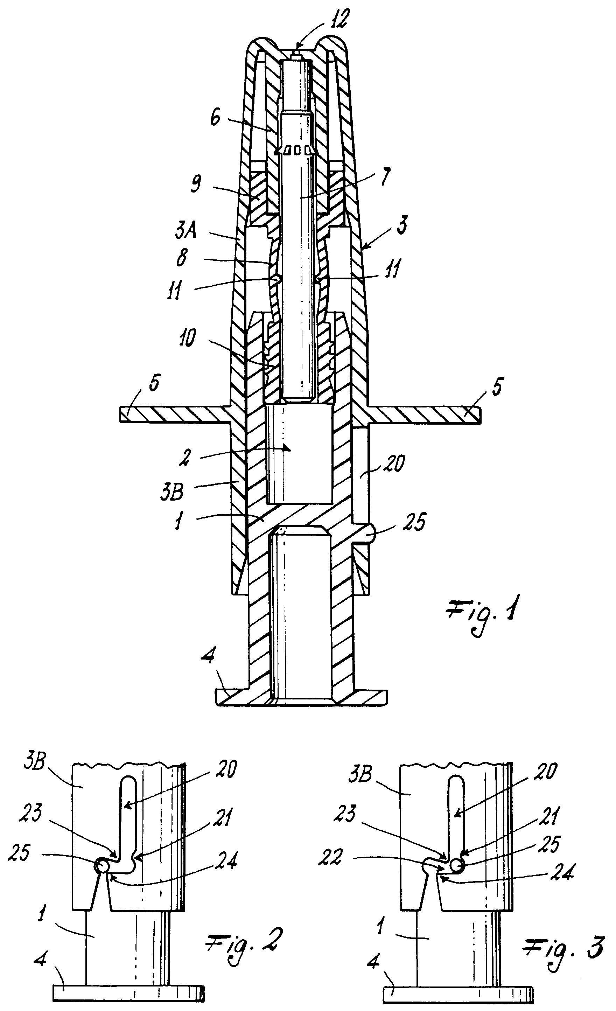

Said body 1 has an elongated shape and it delimits the above said chamber 2 which is open in correspondence of the upper end (with respect to Figure 1) of the body itself, whose lower end has a seat 4 for the thumb of one hand by which the dispenser is actuated.

Said body 3 consists of an elongated hollow element 3A having a rounded profile (as it can be clearly seen from Figure 1) from whose lower end (with respect to said Figure 1) it externally protrudes a wing 5 whereon two fingers of a hand can lean, and whose thumb is leant on the seat 4, at the moment of the dispenser actuation: in the inside of the hollow element 3A, it extends a tubular wall 6, wherein it is inserted and retained the upper end of a cylindrical elongated stem 7 which extends beyond and under the lower edge of said tubular wall and whose other end, namely the lower end of said stem, extends and is slidable into said chamber 2, as it can be clearly seen from Figure 1.

The stem 7 is surrounded by a tubular sleeve 8, 9, 10 made of rubber or of other suitable resiliently yielding material. The portion of the sleeve upper end 9 is mounted and steadily retained on the lower end of said tubular wall 6, while the portion of the lower end thereof is internal to said chamber 2 and it is seal slidable on the cylindrical surface of said chamber.

The intermediate portion 8 of the sleeve consists of a resiliently flexible continuous wall wherefrom it extends towards the inside a continuons annular rib 11 which is resiliently pushed on said stem 7, making seal on it: consequently the intermediate portion 8 of the sleeve takes a "barrel" shape, namely its longitudinal sections are arch shaped, as it can be clearly seen from Fig. 1.

It can be noticed from the drawing that, among the external surface of said stem 7 and the internal surfaces of the sleeve 8, 9, 10 respectively, a continuous passage is delimited (not indicated with reference numbers in the drawing, but provided all around said stem) which is closed, at one of its intermediate positions, by said rib 11 which is kept pressed on said stem 7 by the resilient action of the portion 8 of the sleeve. Said passage opens in correspondence of the upper end 3A of said body 3, where the discharge nozzle 12 is provided wherefrom the liquid drug in atomized form can be discharged.

The above continuous passage can be made for instance, getting one or more grooves in the lower portion and in the upper one of said stem 7 or providing a series of projections in the inside of the portions 9 and 10 of the sleeve.

A protecting cap (not represented in the drawing) can be mounted and snap-retained on the body 3, said cap having to be obviously removed before using the nasal dispenser.

Reference be now made also to Figures 2 and 3. It can be therefrom seen that in the lower part 3B of the body 3 it is got one elongated and shaped window one of whose parts 20 extends longitudinally and has, near its lower portion, a relief 21 in correspondence of which there extends a substantially horizontal part 22 of the window delimited by two projections indicated by the numbers 23 and 25 respectively.

Radially towards the inside, from the body 1 it extends a stem 25 which is inserted and is slidable in the above mentioned window parts 22 and 20.

In the phase of preparing the dispenser, at first the body 1 is moved into the body 3, making the stem 25 slide in the window or slit at the lower end 3B of the body 3, till said stem snap-passes beyond the relief 24, disposing itself and being retained in a tight manner in the first horizontal part 22 of the window, being therein blocked between the projections 23 and 24 (Fig. 2).

Obviously before mounting the body 3 on the body 1, the desired amount of the liquid drug is inserted into the chamber 2, being said drug destined to be dispensed by the dispenser.

It has to be noticed that when in the phase of the dispenser mounting, the stem 7 and the lower portion 10 of the sleeve 8, 9, 10 are moved into the chamber 2: the air therein present upon the surface of the liquid already introduced in said chamber, passes into the free space between said stem and the portion 10 of the sleeve and causes a ball-like swelling of the portion 8 with the consequent lifting of the rib 11 away from said stem 7, in such a way that the air can be discharged towards the outside through the nozzle 12.

When the movement of introducing the stem and the sleeve is ceased, the pressure within the chamber lowers, reaching an equilibrium value, and the rib 11 resiliently and automatically makes back seal on the surface of the stem 7.

This being made, the dispenser is ready for the use.

It has to be noticed that in said conditions, unitentional dispensing of the liquid contained in the chamber 2 is not attained, said liquid remaining hermetically closed in the chamber itself, the stem 25 being retained in the position depicted in Figure 2.

Supposition be now made wanting to use the dispenser: catching with two hands the body 3 and the lower part of the dispenser respectively, a rotation is caused, of the one in respect to the other, around their axis, in such a way that the stem 25 snap-passes the projection 23, disposing itself in the position shown in Figure 3, wherein it is retained between said projection 23 and the projection 21.

The thumb of one hand is then lent on the free end 4 of the body 1 and the forefinger and the medium finger of the same hand are lent on the wings 5 exerting a high pressure which causes the snap-passing of the stem 25 beyond the holding edge 21, thus transmitting a pressure, having since from the start already a very high value to the liquid contained in the chamber 2.

Said liquid after having passed in the open space between the stem and the facing surface of the portion 10 of the sleeve, causes the buckling or swelling towards the outside of the intermediate portion 8 of such sleeve and then the lifting of the annular rib 11 away from the surface of the stem 7, thus allowing the ejection of the liquid drug which passes from the chamber 2 beyond the annular rib 11, crosses the space delimited among the external surface of the stem and the internal surfaces of the sleeve and of the tubular element 6 and discharges under pressure and in finely atomized form through the discharging nozzle 12, to be inhaled in the nostril of the nose of the utilizer.

It can be noticed that the push exerted on the lower surface of the portion 10 of the sleeve by the liquid under pressure, contributes to arch towards the outside the intermediate rounded portion 8 of the sleeve itself and helps therefore the lifting of the rib 11 away from the surface of the stem 7, thus favouring the expulsion of the liquid from the chamber 2.

The structure of the nasal dispenser is rather simple and the cost thereof is low as it can be easily understood by what it has been above described; the liquid in the chamber 2 remains completely isolated before the dispenser use and, during its utilization, the dispensing occurs at a very high pressure of a predetermined value, thus avoiding every form of dropping of the liquid outside of the discharging nozzle.

It is also evident that the structure of the dispenser can be carried out in different forms but equivalent to that one depicted in the drawings. For instance the retaining means of the stem 25, instead of being made up by the relief 21 and by the projections 23 and 24, can consist each of a thin wall or membrane carried out by molding together with the body 3, each of such walls or membranes being suitable to hold the stem 25 and being broken (in such a way that said stem can snap-pass beyond it) when said stem is pushed onto said membrane with a force sufficient to break the membrane itself.

## Claims

1. Manually actuated nasal dispenser for dispensing dosed amounts of atomized liquid drugs, comprising a first body (1) having a cylindrical chamber (2) for containing at least one liquid drug dose and a second body (3) slidingly mounted on said first body and having a nozzle (12) for drug dispensing, said first body (1) having an elongated shape and delimiting said chamber which is open at one end of said first body itself whose other end has a seat (4) for the resting of a finger of one hand, and said second body (3) consisting of a hollow elongated element (3A, 3B) of rounded shape from which at least one shaped element (5) externally extends for the resting of at least another finger of said hand and into which a tubular wall (6) extends to which a piston slidable within said chamber (2) is integral, said nozzle (12) being provided in said hollow element (3A) in correspondence of a free end thereof, one end of an elongated cylindrical stem (7) being inserted and retained within said tubular wall (6), said stem (7) extending beyond the free edge of said tubular wall (6) and having its other end extending into said chamber (2), said piston comprising a tubular sleeve (8, 9, 10) made of resiliently yielding material superimposed to said stem (7), one end portion (9) of said sleeve being mounted on said tubular wall (6) while the other end portion (10) of the same sleeve extends and is seal slidable in said chamber (2), the intermediate portion of said sleeve (8, 9, 10) consisting of a continuos wall (8) resiliently flexible, characterized in that said end portion (9) of the sleeve (8, 9, 10) mounted on the tubular wall (6) is restrained thereon, and in that at least one annular continuous rib (11) projects from said continuous wall (8) towards the inside, said rib (11) being resiliently pushed on the outer cylindrical surface of said stem (7) by the resilient action of said continuous wall (8) to seal on it in rest condition of the dispenser, at least one continuous passage being formed between the internal surface of said sleeve (8, 9, 10) and respectively the external surface of said stem (7), such passage being open at its ends in correspondence of said nozzle (12) and respectively of said chamber (2) and being seal intercepted, in an intermediate position, by said rib (11) protruding from said intermediate portion (8) of said sleeve, said rib being moved away from said stem surface when liquid pressure in said chamber due to actuation by the user causes said continuous wall (8) to be outwardly deformed.

2. Nasal dispenser according to claim 1, characterized in that said first and said second bodies (1, 3) have shaped portions (24, 25) cooperating among themselves to prevent the free end of said stem (7) from disconnecting from said chamber (2).

3. Nasal dispenser according to claims 1 and 2 characterized in that retaining means (21, 24, 25) are provided which can be snap-passed upon manual actuation of said dispenser for retaining said first and second bodies (1, 3) in the rest position preliminary to dispensing.

## Patentansprüche

1. Handbetätigter Nasenspender zum Abgeben dosierter Mengen zerstäubter, flüssiger Medikamente, mit einem ersten Gehäuse (1), das eine zylindrische Kammer (2) zur Aufnahme mindestens einer Dosis eines flüssigen Medikaments aufweist, und einem zweiten Gehäuse (3), das verschieblich am genannten ersten Gehäuse angebracht ist und eine Düse (12) zur Medikamentenabgabe aufweist, wobei das genannte erste Gehäuse (1) eine längliche Form aufweist und die genannte Kammer begrenzt, die am einen Ende des genannten ersten Gehäuses selbst offen ist, dessen anderes Ende einen Sitz (4) zum Auflegen eines Fingers einer Hand aufweist, das genannte zweite Gehäuse (3) aus einem hohlen, länglichen Element (3A, 3B) mit abgerundeter Form besteht, von dem aus sich mindestens ein geformtes Element (5) nach außen zum Auflegen mindestens eines anderen Fingers der genannten Hand erstreckt und in welches hinein sich eine rohrförmige Wand (6) erstreckt, mit der ein Kolben fest verbunden ist, der innerhalb der genannten Kammer (2) verschieblich ist, die genannte Düse (12) im genannten hohlen Element (3A) in Übereinstimmung mit dessen freiem Ende vorgesehen ist, ein Ende eines länglichen, zylindrischen Schaftes (7) in das Innere der genannten rohrförmigen Wand (6) eingeführt und dort gehalten ist, der genannte Schaft (7) sich über die freie Kante der genannten rohrförmigen Wand (6) hinaus erstreckt und sich mit seinem anderen Ende in die genannte Kammer (2) erstreckt, der genannte Kolben eine rohrförmige Manschette (8, 9, 10) aufweist, die aus federnd nachgiebigem Material hergestellt ist, das den genannten Schaft (7) umgibt, ein Endabschnitt (9) der genannten Manschette an der genannten rohrförmigen Hülse (6) angebracht ist, während der andere Endabschnitt (10) derselben Manschette sich in der genannten Kammer (2) erstreckt und dichtend verschieblich ist, und der Zwischenabschnitt der genannten Manschette (8, 9, 10) aus einer durchgehenden Wand (8) besteht, die federnd flexibel ist, dadurch **gekennzeichnet,** daß der genannte Endabschnitt (9) der Manschette (8, 9, 10), der an der rohrförmigen Wand (6) angebracht ist, an dieser unter Spannung festsitzt, und daß mindestens eine ringförmige, durchgehende Rippe (11) von der genannten durchgehenden Wand (8) zur Innenseite hin vorsteht, wobei die genannte Rippe (11) federnd auf die äußere, zylindrische Oberfläche des genannten Schaftes (7) durch die federnde Wirkung der genannten durchgehenden Wand (8) aufgedrückt ist, um hieran im Ruhezustand des Spenders abzudichten, mindestens ein durchgehender Kanal zwischen der Innenfläche der genannten Manschette (8, 9, 10) bzw. der Außenfläche des genannten Schaftes (7) gebildet ist, ein solcher Kanal an seinen Enden in Übereinstimmung mit der genannten Düse (12) bzw. der genannten Kammer (2) offen und in einer Zwischenlage durch die genannte Rippe (11) dichtend unterbrochen ist, die vom genannten mittleren Abschnitt (8) der genannten Manschette vorsteht, und die genannte Rippe von der genannten Schaftfläche wegbewegt wird, wenn der Flüssigkeitsdruck in der genannten Kammer infolge der Betätigung durch den Benutzer die genannte durchgehende Wand (8) veranlaßt, nach außen verformt zu werden.

2. Nasenspender nach Anspruch 1, dadurch **gekennzeichnet,** daß das genannte erste und das genannte zweite Gehäuse (1, 3) geformte Abschnitte (24, 25) aufweisen, die miteinander zusammenwirken, um die freien Enden des genannten Schaftes (7) daran zu hindern, sich aus der Verbindung mit der genannten Kammer (2) zu lösen.

3. Nasenspender nach Anspruch 1 und 2, dadurch **gekennzeichnet,** daß Haltemittel (21, 24, 25) vorgesehen sind, die infolge der Betätigung des genannten Spenders von Hand rastend bewegt werden können, um das genannte erste und zweite Gehäuse (1, 3) vor der Ausgabe in der Ruhelage zu halten.

## Revendications

1. Pulvérisateur nasal actionné à la main pour distribuer des doses d'un médicament liquide atomisé, ce pulvérisateur comprenant un premier corps (1) muni d'une chambre cylindrique (2) destinée à contenir au moins une dose de médicament liquide, et un deuxième corps (3) monté pour coulisser sur le premier corps et muni d'une buse (12) de distribution du médicament, ledit premier corps (1) étant de forme allongée et délimitant la chambre qui est ouverte a une extrémité dudit premier corps même, dont l'autre extrémité présente un siège (4) pour poser un doigt d'une main, le deuxième corps (3) étant constitué par un élément allongé creux (3A, 3B) de forme arrondie, duquel s'étend à l'extérieur au moins un élément profilé (5) permettant d'y poser au moins un autre doigt de la main, et dans lequel s'étend une paroi tubulaire (6) avec laquelle est solidarisé un piston qui coulisse dans la chambre (2), la buse (12) étant prévue dans l'élément creux (3A) en correspondance avec une de ses extrémités libres, une extrémité d'une tige cylindrique allongée (7) étant insérée et maintenue dans la paroi tubulaire (6), la tige (7) s'étendant au-delà du bord libre de ladite paroi tubulaire (6), et l'autre extrémité de ladite tige s'étendant dans la chambre (2), le piston comprenant une gaine tubulaire (8, 9, 10) réalisée dans une matière souple et molle superposée sur la tige (7), une portion terminale (9) de la gaine étant montée sur la paroi tubulaire (6), tandis que l'autre portion terminale (10) de la même gaine s'étend et coulisse avec étanchéité dans ladite chambre (2), la portion intermédiaire de la gaine (8, 9, 10) consistant en une paroi continue (8) et déformable élastiquement,
caractérisé en ce que la portion terminale (9) de la gaine (8, 9, 10) qui est montée sur la paroi tubulaire (6) est serrée sur celle-ci, et en ce qu'au moins une nervure annulaire continue (11) fait saillie vers l'intérieur de la paroi continue (8), ladite nervure (11) étant poussée élastiquement sur la face cylindrique extérieure de la tige (7) par l'action élastique de la paroi continue (8) pour y être maintenue de manière étanche lorsque le pulvérisateur est au repos, au moins un passage continu étant formé respectivement entre la surface interne de la gaine (8, 9, 10) et la face externe de la tige (7), ce passage étant ouvert à ses extrémités en correspondance avec la buse (12) et la chambre (2) resectivement, et obturé de manière étanche dans une position intermédiaire par la nervure (11) qui fait saillie de la portion intermédiaire (8) de la gaine, ladite nervure étant écartée de la surface de la tige lorsque la pression du liquide dans la chambre provoquée par une action de l'utilisateur déforme la paroi continue (8) vers l'extérieur.

2. Pulvérisateur nasal selon la revendication 1,
caractérisé en ce que les premier et deuxième corps (1, 3) présentent des portions profilées (24, 25) qui coopèrent entre elles pour éviter que l'extrémité libre de la tige (7) soit déconnectée de la chambre (2).

3. Pulvérisateur nasal selon la revendication 1 et 2,
caractérisé en ce que sont prévus des moyens de retenue (21, 24, 25) qui permettent un passage brusque lorsqu'on agit manuellement sur le pulvérisateur, ces moyens retenant le premier et le deuxième corps (1, 3) dans la position de repos avant la pulvérisation.
